(19) Europäisches Patentamt
European Patent Office
Office européen des brevets

(11) Veröffentlichungsnummer: **0 160 915**
**A2**

(12) # EUROPÄISCHE PATENTANMELDUNG

(21) Anmeldenummer: 85105197.9

(22) Anmeldetag: 29.04.85

(51) Int. Cl.[4]: C 07 F 9/54
C 07 C 121/48, H 01 B 1/12

(30) Priorität: 11.05.84 DE 3417466

(43) Veröffentlichungstag der Anmeldung: 13.11.85 Patentblatt 85/46

(84) Benannte Vertragsstaaten: CH DE FR GB IT LI NL

(71) Anmelder: BAYER AG
Konzernverwaltung RP Patentabteilung
D-5090 Leverkusen 1 Bayerwerk(DE)

(72) Erfinder: Jonas, Friedrich, Dr.
Krugenofen 15
D-5100 Aachen(DE)

(72) Erfinder: Hocker, Jürgen, Dr.
Eichenweg 6
D-5060 Bergisch-Gladbach 2(DE)

(54) **Schmelzbare, elektrisch leitfähige TCNQ-Komplexe.**

(57) Die Erfindung betrifft schmelzbare, elektrisch leitfähige TCNQ-Komplexe, Verfahren zu ihrer Herstellung, und ihre Verwendung zur Herstellung elektrisch leitender Überzüge auf Substraten durch Aufschmelzen.

EP 0 160 915 A2

BAYER AKTIENGESELLSCHAFT 5090 Leverkusen, Bayerwerk

Konzernverwaltung RP
Patentabteilung G/Ke-c

# Schmelzbare, elektrisch leitfähige TCNQ-Komplexe

Komplexsalze aus dem 7.7.8.8-Tetracyano-p-chinodimethananion (TCNQ)$^{-\cdot}$

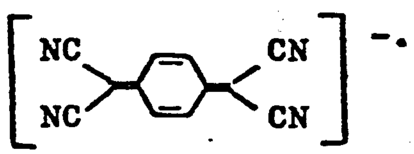

neutralem 7.7.8.8-Tetracyano-p-chinodimethan (TCNQ) und anorganischen oder organischen Kationen sind als elektrisch leitende Verbindungen bekannt und können durch Umsetzung von TCNQ mit organischen Kationjodiden hergestellt werden /J.Am. Chem. Soc. 84, 3374 - 3387 (1962)/, z.B. gemäß

$$4\ TCNQ + 3M^{+}J^{-} \longrightarrow 2\ M^{+} .\ TCNQ^{-} .\ TCNQ + M^{+}J_{3}^{-}$$

Reaktionsschema I

Hierbei wird ein TCNQ-Molekül durch Jodid zum TCNQ Radikal-Anion unter Freisetzung von Jod reduziert.

Ein anderes Verfahren besteht in der Umsetzung von stickstoffhaltigen Heteroaromaten oder tertiären Aminen mit "$H_2$TCNQ" (vgl. Reaktionsnehmer II) und TCNQ, z.B. gemäß

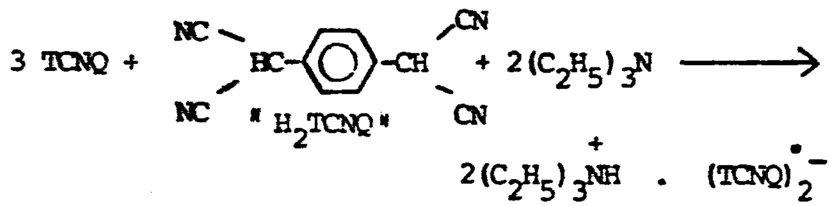


Reaktionsschema II

Die Verarbeitung dieser Komplexe bei Temperaturen oberhalb ihres Schmelzpunktes bereitet Schwierigkeiten, da die bisher bekannten Verbindungen sich bereits am Schmelzpunkt oder wenig oberhalb des Schmelzpunktes zersetzen (s. Tab. 1).

Es wurde gefunden, daß Komplexe aus TCNQ und organischen Phosphoniumionen längere Zeit im geschmolzenen Zustand gehalten werden können, ohne daß ihre elektrische Leitfähigkeit nach dem Erkalten wesentlich vermindert ist.

Gegenstand der Erfindung sind daher TCNQ-Komplexe der allgemeinen Formel I

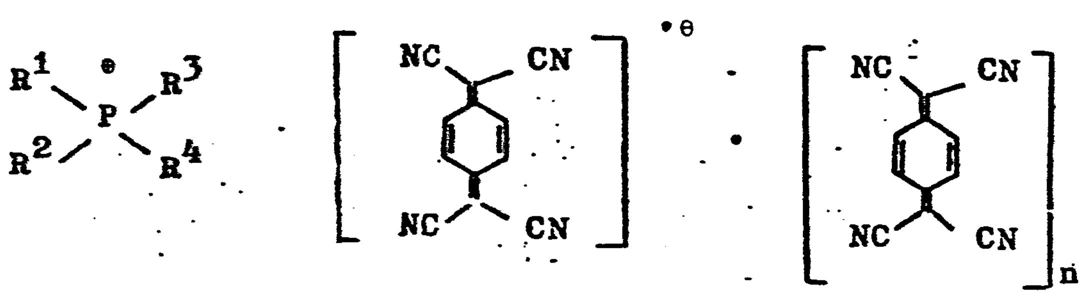

(I)

worin

n 2, 1 oder 0 (bevorzugt 1 oder 0) bedeutet,

$R^1$, $R^2$, $R^3$, gleich oder verschieden, für einen gegebenenfalls substituierten geradkettigen oder verzweigten Alkylrest mit 1 bis 30 C-Atomen, einen gegebenenfalls substituierten Cycloalkylrest mit 5 bis 12 C-Atomen, einen gegebenenfalls substituierten Arylrest mit 6 bis 14 C-Atomen oder einen heterocyclischen Rest mit 4 bis 12 C-Atomen stehen und

$R^4$ einen gegebenenfalls substituierten geradkettigen oder verzweigten Alkylrest mit 3 bis 30 C-Atomen, einen gegebenenfalls substituierten Cycloalkylrest mit 5 bis 12 C-Atomen, einen gegebenenfalls substituierten Arylrest mit 6 bis 14 C-Atomen oder einen heterocyclischen Rest mit 4 bis 12 C-Atomen bedeutet.

Bevorzugt sind Verbindungen der Formel I, in denen $R^1$=$R^2$=$R^3$=Aryl, insbesondere Phenyl, und $R^4$= $C_3$ bis $C_{24}$-Alkyl ist.

Als Beispiele für $R^1$, $R^2$, $R^3$ seien formelmäßig angegeben:

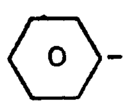 , 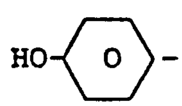 , 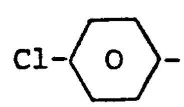 ,

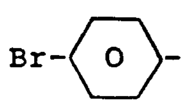 , 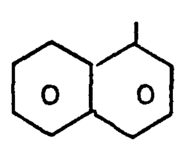 , 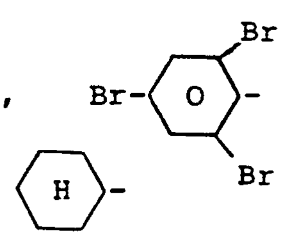 ,

$CH_3$-$CH_2$-, $CH_3$-$CH_2$-$CH_2$-$CH_2$-,

Als Beispiele für $R^4$ seien formelmäßig angegeben:

$CH_3-CH_2-CH_2-$

$CH_3-CH_2-CH_2-CH_2-$

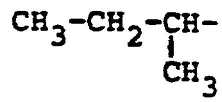

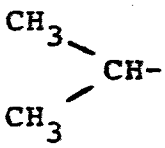

$CH_3-CH_2-CH_2-CH_2-CH_2-$

$CH_3-CH_2-CH_2-CH_2-CH_2-CH_2-$

$CH_3-CH_2-CH_2-CH_2-CH_2-CH_2-CH_2-$

$CH_3-CH_2-CH_2-CH_2-CH_2-CH_2-CH_2-CH_2-CH_2-$

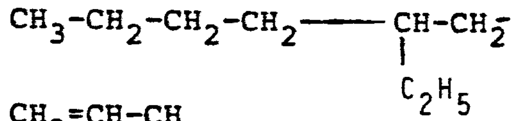

$CH_2=CH-CH_2$

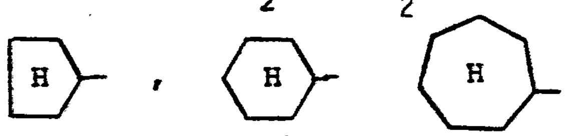

Besonders bevorzugt sind Verbindungen der Formel I, in denen $R^1 = R^2 = R^3 =$ Phenyl und $R^4 = C_3$ bis $C_{12}$-Alkyl ist, da diese Verbindungen niedrige Schmelzpunkte bei hohen Zersetzungstemperaturen aufweisen.

Besonders bevorzugt sind somit TCNQ-Komplexsalze der Formel

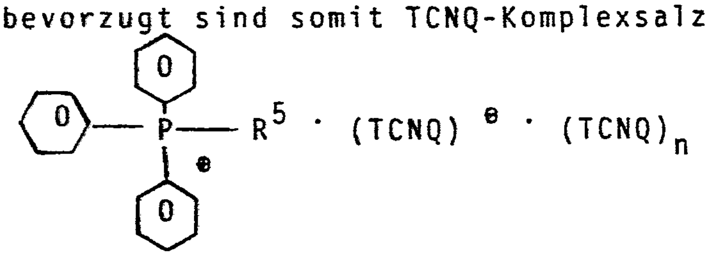


worin $R^5 = C_3 - C_{24}$ Alkyl und
$n = 2, 1$ oder 0 ist.

Die erfindungsgemäßen Komplexe können vorteilhaft durch Umsetzung von Lösungen organischer Phosphoniumjodide mit einer Lösung von TCNQ in organischen Lösungsmitteln bei Temperaturen unterhalb 150°C, bevorzugt von 50 - 150°C, hergestellt werden.

Geeignete Phosphoniumjodide entsprechen der Formel

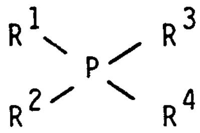 $J^-$ , worin $R^1$ bis $R^4$ die bereits genannte Bedeutung haben.

Beispiele für geeignete Phosphoniumjodide sind Triphenylmethylphosphoniumjodid, Triphenylethylphosphoniumjodid, Triphenylbutylphosphoniumjodid, Triphenyloctylphosphoniumjodid.

Geeignete organische Lösungsmittel sind z.B. Halogenkohlenwasserstoffe, z.B. Methylenchlorid, Chloroform, Tetrachlorkohlenstoff, 1,2-Dichlorethan, 1,1,2-Trichlorethan; Acetonitril; Alkohole, z.B. Methanol, Ethanol, Isopropanol; aliphatische Ketone, z.B. Aceton, Methylethylketon; acyclische und cyclische Ether, z.B. Diethylether, Tetrahydrofuran. Bevorzugt sind hierbei Lösungsmittel mit niedrigem Siedepunkt (unter 100°C), um eine thermische Schädigung des gebildeten Komplexes zu vermeiden.

Die Reaktionspartner werden bevorzugt im Verhältnis 1 Mol TCNQ zu 0,5 bis 1,5 Mol Phosphoniumjodid eingesetzt.

Aufgrund ihres günstigen Schmelz- und Zersetzungsverhaltens können die Verbindungen der Formel I zur Herstellung elektrisch leitender Überzüge auf Substraten durch Aufschmelzen verwendet werden.

In vorteilhafter Weise können auch Gemische verschiedener TCNQ-Komplexe, die mindestens eine Verbindung der Formel I enthalten, aus der Schmelze zu leitenden Überzügen verarbeitet werden. Diese Überzüge können gegebenenfalls auch stabilisierende, die Haftung verbessernde bzw. farbgebende Zusätze enthalten.

Als geeignete Substrate seien genannt: Glas, Metalle, Metalloxide, organische Polymere.

Das Beschichten der Substrate kann in der Weise erfolgen, daß die Substrate auf Temperaturen oberhalb des Schmelzpunktes der TCNQ-Komplexe der Formel I aufgeheizt werden und anschließend die TCNQ Komplexe aufgestreut werden.

Das Aufschmelzen der TCNQ-Komplexe kann auch unter Schutzgasen wie z.B. Wasserstoff, Stickstoff, Argon oder Helium bzw. im Vakuum durchgeführt werden.

Bei einem anderen Verfahren geht man so vor, daß die TCNQ-Komplexe der Formel I bei Raumtemperatur auf die zu beschichtenden Substrate aufgebracht und anschließend in einem vorgeheizten Ofen aufgeschmolzen werden.

Beide Verfahren führen zu elektrisch leitfähigen, gut haftenden Überzügen.

Die so hergestellten Überzüge finden Verwendung in der Elektronikindustrie.

Beispiel 1

20,4 g TCNQ in 1.000 ml Acetonitril werden bei Rückfluß mit einer 70°C heißen Lösung aus 35 g Triphenylmethylphosphoniumjodid in 400 ml Acetonitril versetzt und 1 Stunde bei 50°C gerührt. Anschließend wird abgekühlt und der über Nacht auskristallisierte Niederschlag abgesaugt und getrocknet.
Ausb.: 31,8 g ≙ 93 % d.Th.
Komplex der Zusammensetzung:

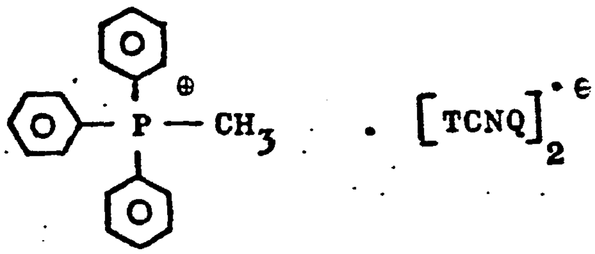

Beispiel 2

Wie in Beispiel 1 beschrieben, werden 4,1 g TCNQ in 120 ml Acetonitril und 7,0 g Triphenylethylphosphoniumjodid in 100 ml Acetonitril zur Reaktion gebracht und aufgearbeitet.
Ausb.: 6,1 g ≙ 87 % d.Th.
Komplex der Zusammensetzung:

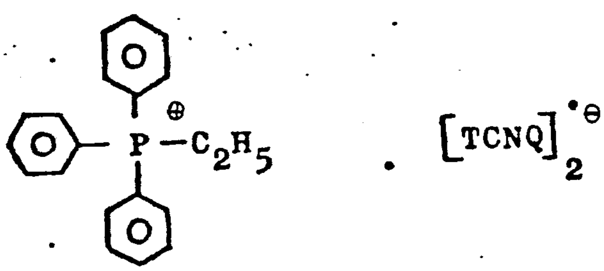

Beispiel 3

Wie in Beispiel 1 beschrieben, werden 4,1 g TCNQ in 300 ml Acetonitril und 6,5 g 2-Propyl-triphenylphosphoniumjodid zur Reaktion gebracht und aufgearbeitet.
Ausbeute: 6,4 g ≙ 90 % d.Th.
Komplex der Zusammensetzung:

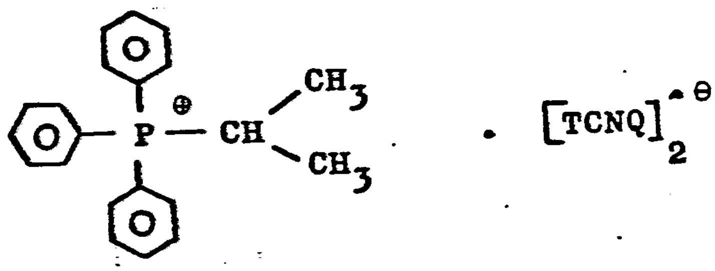

Beispiel 4

Wie in Beispiel 1 beschrieben, werden 4,1 g TCNQ in 300 ml Acetonitril und 6,7 g n-Butyl-triphenylphosphoniumjodid in 100 ml Acetonitril zur Reaktion gebracht und aufgearbeitet.
Ausbeute: 5,9 g ≙ 81 % d.Th.
Komplex der Zusammensetzung:

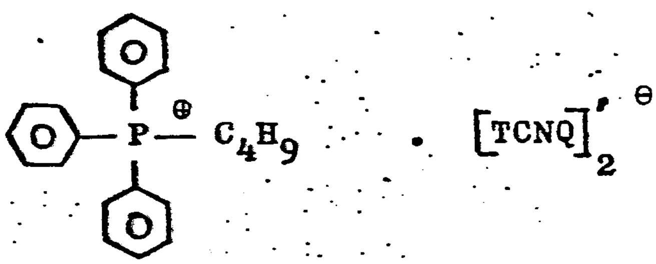

Beispiel 5

Wie in Beispiel 1 beschrieben, werden 4,1 g TCNQ in 300 ml Acetonitril und 10,0 g n-Octyl-triphenylphosphoniumjodid in 100 ml Acetonitril zur Reaktion gebracht und aufgearbeitet.
Ausbeute: 6,1 g ≙ 78 % d.Th.
Komplex der Zusammensetzung:

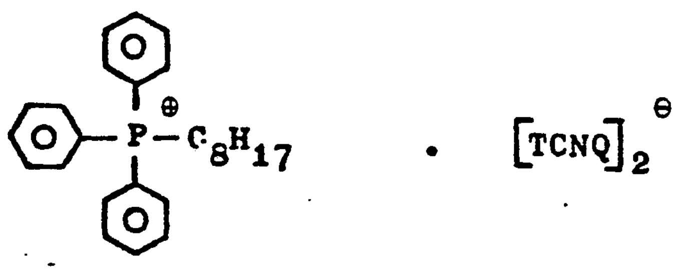

Schmelz- und Zersetzungspunkte der Verbindungen gemäß Beispiel 1 bis 5 siehe Tabelle 1.

Tabelle 1 DSC-Messungen

(Aufheizrate 80 K/min)

| Verbdg. nach Beispiel | $R^4$ | Schmelzpunkt °C | Zers. punkt °C |
|---|---|---|---|
| 1 | $-CH_3$ | 256 | 300 |
| 2 | $-C_2H_5$ | 244 | 310 |
| 3 | $i-C_3H_7$ | 245 | 317 |
| 4 | $-C_4H_9$ | 216 | 318 |
| 5 | $-C_8H_{17}$ | 182 | 306 |
| | * | 270 | 285 |

Vergleich

*= 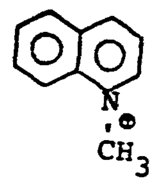 $\cdot [TCNQ]_2^{\ominus}$

Die Schmelz- bzw. Zersetzungspunkte wurden mittels Differential-Scanning-Calorimetrie ermittelt. Angegeben sind die Temperaturen der Maxima der endothermen Schmelz- bzw. exothermen Zersetzungsphase.

Die thermische Stabilität der erfindungsgemäßen Komplexe ist aus Tabelle 2 ersichtlich.

Die Komplexe wurden in Glasgefäßen für die angegebene Zeit auf die angegebene Temperatur erhitzt und anschliessend auf Raumtemperatur abgekühlt. Die Leitfähigkeit der reinen Komplexe $\delta°$ vor bzw. nach dem Erhitzen wurde mittels Zweielektrodenmessung bestimmt.

Tabelle 2

| Komplexe nach Beispiel | T [°C] | $\delta^{\circ}$ [S/cm] | Min. | $\delta$ [S/cm] | Min. | $\delta$ [S/cm] | Min. | $\delta$ [S/cm] |
|---|---|---|---|---|---|---|---|---|
| 1 | 270 | $8 . 10^{-3}$ | 1 | $1 . 10^{-3}$ | 3 | $2 . 10^{-4}$ | 5 | $6 . 10^{-6}$ |
| 2 | 260 | $1 . 10^{-2}$ | 1 | $2 . 10^{-3}$ | 3 | $6 . 10^{-4}$ | 5 | $5 . 10^{-8}$ |
| 3 | 260 | $6 . 10^{-3}$ | 1 | $5 . 10^{-3}$ | 3 | $5 . 10^{-3}$ | 5 | $1 . 10^{-4}$ |
| 4 | 230 | $7 . 10^{-3}$ | 1 | $4 . 10^{-3}$ | 3 | $3 . 10^{-3}$ | 5 | $7 . 10^{-4}$ |
| 5 | 200 | $9 . 10^{-3}$ | 1 | $7 . 10^{-3}$ | 3 | $6 . 10^{-3}$ | 5 | $2 . 10^{-3}$ |
| Vergleich | 280 | $1 . 10^{-1}$ | 1 | $5 . 10^{-8}$ | 3 | - | 5 | - |

Beispiel 6

Eine Glasplatte wurde in einem Ofen auf 320°C aufgeheizt. Anschließend wurde eine ca. 0.5 mm dicke Schicht des Komplexes nach Beispiel 1 aufgestreut. Der Komplex schmilzt hierbei und bildet nach dem Abkühlen einen harten, zusammenhängenden, elektrisch leitfähigen Überzug.

Beispiel 7-10

Es wurde wie in Beispiel 6 vorgegangen unter Verwendung der Komplexe nach Beispiel 2 - 5 und unter Veränderung der Temperaturen:

| Komplex nach Beispiel | T [°C] |
|---|---|
| 2 | 280 |
| 3 | 280 |
| 4 | 250 |
| 5 | 210 |

In allen Fällen wurden elektrisch leitfähige Überzüge erhalten.

Beispiel 11-15

Auf Glasplatten wurden ca. 0,5 mm dicke Schichten der Komplexe 1 - 5 aufgestreut und anschließend im Warmluftofen bei der angegebenen Temperatur aufgeschmolzen (Dauer 4 bis 6 Minuten)

| Komplex nach Beispiel | T [°C] |
|---|---|
| 1 | 280 |
| 2 | 280 |
| 3 | 270 |
| 4 | 240 |
| 5 | 210 |

In allen Fällen wurden elektrisch leitfähige Überzüge erhalten.

Patentansprüche

1. TCNQ-Komplexsalze der allgemeinen Formel I

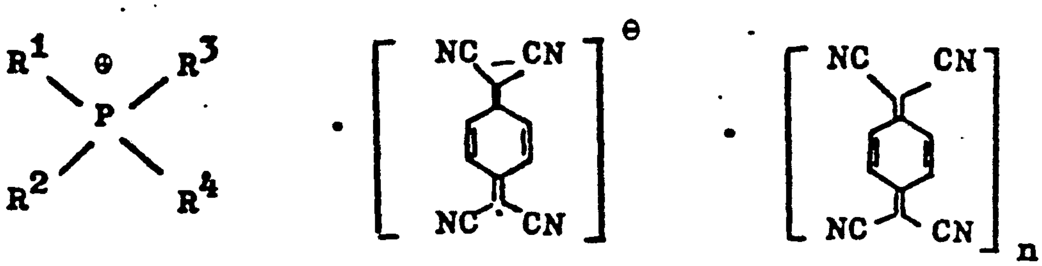

(I)

worin

n 2, 1 oder 2 bedeutet,

$R^1$, $R^2$, $R^3$, gleich oder verschieden, für einen gegebenenfalls substituierten geradkettigen oder verzweigten Alkylrest mit 1 bis 30 C-Atomen, einen gegebenenfalls substituierten Cycloalkylrest mit 5 bis 10 C-Atomen, einen gegebenenfalls substituierten Arylrest mit 6 bis 14 C-Atomen oder einen heterocyclischen Rest mit 4 bis 12 C-Atomen steht und $R^4$ einen gegebenenfalls substituierten geradkettigen oder verzweigten Alkylrest mit 3 - 30 C-Atomen, einen gegebenenfalls substituierten Cycloalkylrest mit 5 bis 12 C-Atomen, einen gegebenenfalls substituierten Arylrest mit 6 bis 14 C-Atomen oder einen heterocyclischen Rest mit 4 bis 12 C-Atomen bedeutet.

2. TCNQ-Komplexsalze der allgemeinen Formel II,

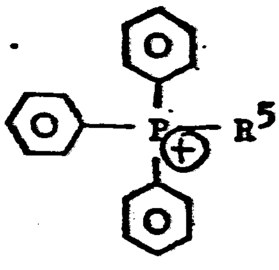

$\cdot\ (TCNQ)^{\ominus} \cdot (TCNQ)_n$

worin

n 2, 1 oder 0 ist und
$R^5$ für einen linearen oder verzweigten
$C_3$ bis $C_{24}$-Alkylrest steht.

3. Verwendung von TCNQ-Komplexsalzen gemäß Anspruch 1 zur Herstellung elektrisch leitfähiger Überzüge durch Aufschmelzen auf Substraten.